(19)

Europäisches
Patentamt
European
Patent Office
Office européen
des brevets

(11) **EP 2 623 967 A1**

(12) **EUROPEAN PATENT APPLICATION**

(43) Date of publication:
    **07.08.2013 Bulletin 2013/32**

(51) Int Cl.:
    **G01N 25/20** (2006.01)          **C12Q 1/04** (2006.01)

(21) Application number: **12382043.3**

(22) Date of filing: **06.02.2012**

| | |
|---|---|
| (84) Designated Contracting States:<br>**AL AT BE BG CH CY CZ DE DK EE ES FI FR GB**<br>**GR HR HU IE IS IT LI LT LU LV MC MK MT NL NO**<br>**PL PT RO RS SE SI SK SM TR**<br>Designated Extension States:<br>**BA ME** | (72) Inventors:<br>• **Lago Rivero, Natividad**<br>  **E-36202 Vigo - Pontevedra (ES)**<br>• **Arias Santos, Isaac**<br>  **E-36202 Vigo - Pontevedra (ES)**<br>• **Legido Soto, José Luis**<br>  **E-36310 Vigo - Pontevedra (ES)** |
| (71) Applicants:<br>• **Fundación Biomédica del Complexo Hospitalario**<br>  **Universitario de Vigo**<br>  **36211, Vigo (ES)**<br>• **Servizo Galego De Saúde (Sergas)**<br>  **15703 Santiago de Compostela , A Coruña (ES)**<br>• **Universidad de Vigo**<br>  **36310 Vigo (ES)** | (74) Representative: **ABG Patentes, S.L.**<br>  **Avenida de Burgos, 16D**<br>  **Edificio Euromor**<br>  **28036 Madrid (ES)** |

(54) **Method and apparatus for detecting microorganisms**

(57)     The invention relates to a method and apparatus for detecting and/or identifying the presence of microorganisms in samples, including the following stages: obtaining a database which establishes a correlation between at least one parameter and a plurality of microorganisms, among which said microorganism is included; analyzing said sample in a calorimeter which supplies an electrical signal proportional to the energy given off by said sample; obtaining a curve which relates said electrical signal with respect to time; extracting from said curve at least one parameter indicative of the behavior of said microorganism; and comparing said parameter extracted from said curve with said parameter contained in said database to establish a conclusion as to the presence of said microorganism in said sample. The stage of extracting at least one parameter indicative of the behavior of said microorganism from said curve can include the division of said curve into at least two sections and the mathematical adjustment of each of said sections to two different mathematical equations, at least one of said sections adjusted to an exponential equation and/or at least another one of said sections is adjusted to a polynomial equation.

EP 2 623 967 A1

**Description**

**Technical Field of the Invention**

The present invention is aimed at a method and apparatus for detecting and/or identifying the presence of microorganisms in samples.

Microorganisms have been key players in historical events such as the fall of the Roman Empire or the conquest of the New World. In 1347, the plague swept across Europe with brutal force: it killed one third of the population in four years. In the following 80 years, this disease continued its assault until eliminating 75% of the European population. The effect of the plague was so enormous that some historians believe it changed European culture, clearing the way for the Renaissance.

The importance of microorganisms cannot be overestimated. In terms of number and mass, they amply exceed any other group of organisms on the planet; they are furthermore found everywhere and they largely contribute to the working of the biosphere. Society in general benefits from them because they are necessary for making bread, cheese, antibiotics, vaccines, vitamins, enzymes and many other important products.

Very shortly after birth, humans are colonized by microorganisms, and some of them will live in permanent symbiosis with their host on the skin, in the digestive tract, upper respiratory tracts, ears and many other tissues, constituting normal microbial flora. Some participate in fundamental biochemical processes for human life. Therefore, a balance is established which can be altered in certain situations, causing what is known as infection.

An arsenal of therapeutic drugs is currently available, but to optimize treatment, a method of early diagnosis that allows an early start of treatment and which is adjusted to the sensitivities of the causing microorganism is necessary.

**Background of the Invention**

In the scientific literature dedicated, there are many publications to detecting microorganisms. Traditional methods, typically including growth and culture of the different specimens in favorable media, as well as subsequent ocular, microscopic identification and/or identification by means of chemical or biochemical analysis of the obtained results, generally requires several days of careful laboratory work.

In the last few decades, alternative processes for detecting microorganisms have been developed, with the objective of solving the problem of the need for obtaining results in the shortest time possible. These processes include the use of the microcalorimetry. The use of microcalorimetry measurements in detecting bacterial growth and, in general, in biochemical sample analysis, is based on the fact that bacterial cultures produce an energy exchange derived from their metabolic processes. The amount of heat produced, which is detectable by microcalorimetry, can thus be considered as one of the measurements of metabolic activity. This relationship is known; see for example patent publications US3,552,207, US3,765,237 or US2004/0235086.

Microcalorimetry is, therefore, an experimental technique which allows determining with high sensitivity the exhibited energy as a consequence of any transformation process.

This technique arouses interest in biological sciences because heat flow is strongly related to biological process kinetics and thermodynamics. Heat variations resulting from chemical reactions which take place during metabolism can be used to monitor bacterial growth and to see the influence of external agents.

All living beings exchange heat as a consequence of their metabolism. Heat rate is a suitable measurement of metabolic activity of organisms and their constituent parts, cells and subcellular levels. The heat generated by a single cell is in the range of 1-80 pW. Human connective tissue cells (fibroblasts, adipocytes, etc.) have reported metabolic rates of approximately 25-80 pW per cell. In contrast, microorganisms produce small amounts of heat, of the order of 1-3 pW per cell. Despite bacteria's low amount of heat, exponential replication in culture media allows microcalorimetric detection in a few hours, even when using samples with a low concentration of bacteria (10 CFU/ml).

Microcalorimetry has been used in biology, pharmacology, biotechnology and ecology because of its high sensitivity, precision and simplicity, but use in clinical practice has been very limited.

This technique arouses interest because heat flow is strongly related to biological process kinetics and thermodynamics. In microbiological research, calorimetry is particularly valuable because it allows to see quickly changes in metabolism and growth speed and efficacy in the presence of different agents. It further has the advantage that measurements can be seen in time real.

In recent years this process has gained growing interest. Several studies have been published which suggest the possibility of identifying different microorganisms by means of microcalorimetry, which include, by way of example, the following: Thomas Maskow et al.: Calorimetric real time monitoring of lambda prophage induction, Journal of Virological Methods 168 (2010) 126-132; "The heat is on": Rapid microcalorimetric detection of mycobacteria in culture, Tuberculosis 90 (2010) 57-59; Dai Chunmei et al.: Investigation of anti-microbial activity of catechin on Escherichia coli growth by microcalorimetry, Environmental Toxicology and Pharmacology 30 (2010) 284-288; Weijun Kong et al.: Screening for novel antibacterial agents based on the activities of compounds on metabolism of Escherichia coli: a microcalorimetric study; G. Buttiglieri et al.: Microcalorimetry: A tool to investigate aerobic, anoxic and anaerobic autotrophic and heterotrophic biodegradation, Biochemical Engineering Journal 52 (2010) 25-32; F. Allenberger: Listeria Monocytogenes - Microcalorimetric Investigations Regarding The Antibacterial Efficiency of Chemotherapeutics, Thermochimica Acta 119 (1987) 113-119; Xie Chang-Li et al.: Microcalorimetric Study Of Bacterial Growth, Thermochimica Acta 123 (1988) 33-41, or Natividad Lago Rivero et al.: Microcalorimetric study of the growth of Enterococcus faecalis in an enriched culture medium, J Therm Anal Calorim (2011) DOI 10.1007/s10973-011-2052-1. Patent publications WO2007/010379 and EP1785722 also relate to detecting microorganisms by means of microcalorimetry.

Some of the aforementioned publications show the possibility of identifying certain types of microorganisms or bacteria by analyzing thermograms obtained by microcalorimetry and the progress which these processes entail with respect to traditional analysis methods by growth and culture of said microorganisms is acknowledged. However, despite the idea that under certain conditions the thermogram can come to be a kind of "fingerprint" from which each of the bacteria of interest can be identified, said pieces of the state of the art state that there is a need to develop these results to clear the way for new possibilities for the rapid identification of microorganisms by means of a simple, reliable and easily reproducible process.

Therefore, the main objective of the present invention is to propose a method and apparatus which allow detecting and/or identifying the presence of microorganisms in various types of samples and solve the problems identified up until now by the aforementioned literature. In particular, the objective is for the new process to be capable of performing this detection and identification in a rapid, simple, reliable and reproducible manner.

Other objectives and purposes of the present invention are easily deduced from the advantages provided by the different technical features which will be used to define the present invention as well as from the particular examples for carrying it out which are described below.

## Summary of the Invention

For the purpose of solving the problems described above and others that will be easily deduced from the advantages provided by the different features and embodiments of the present invention described below, the present invention presents a method of detecting and/or identifying a microorganism in a sample as described in claim 1, and an apparatus for detecting and/or identifying a microorganism in a sample according to claim 9. The dependent claims describe particularly advantageous embodiments of the present invention.

Particularly, the present invention presents in a first inventive aspect a method of detecting and/or identifying a microorganism in a sample, including the following stages:

- obtaining a database which establishes a correlation between at least one parameter and a plurality of microorganisms, among which said microorganism is included;
- analyzing said sample in a calorimeter which supplies an electrical signal proportional to the energy given off by said sample;
- obtaining a curve which relates said electrical signal with respect to time;
- extracting from said curve at least one parameter indicative of the behavior of said microorganism; and
- comparing said parameter extracted from said curve with said parameter contained in said database to establish a conclusion as to the presence of said microorganism in said sample.

With the sequence of steps of the aforementioned process, a method which is essentially faster and more reliable than methods known until now is achieved. The authors of the present invention have identified a series of characteristic parameters of each microorganism resulting from the mathematical analysis of the different thermograms. Therefore, by previously performing a process of microcalorimetric measurement of all the microorganisms of interest at different concentrations, a database with all the characteristic parameters can be obtained, such that when analyzing a culture that is in principle unknown, it is no longer necessary to carry out the individualized identification of the thermograms, as has been done until now, but it is sufficient to extract one or several of the parameters of interest in an automated manner and to perform a comparison with the information contained in the database.

The stage of obtaining a database can advantageously consist of performing the following steps:

- preparing a sample of one of the microorganisms included in said plurality of microorganisms;
- analyzing said sample in a calorimeter which supplies an electrical signal proportional to the energy given off by said sample;
- obtaining a curve which relates said electrical signal with respect to time;
- extracting from said curve at least one parameter indicative of the behavior of said microorganism;
- transferring the results of said parameter or parameters together with the identification of the chosen microorganism to an electronic system capable of creating a database which establishes a relationship between both; and
- repeating all the aforementioned steps with each of the microorganisms included in said plurality of microorganisms.

The stage or stages of analyzing the sample in a calorimeter can advantageously also comprise performing the following steps:

- providing a Calvet-type heat conduction calorimeter, provided with at least one thermostatic system and with one detector system formed by two identical thermopiles,
- providing a data acquisition and treatment system;
- providing two cells, an experimental cell and a control cell;
- introducing a portion of culture medium into the experimental cell and introducing a portion of culture medium plus physiological saline, preferably 0.9% sodium chloride, into the control cell;
- introducing both cells in the respective internal enclosures of each of said thermopiles;
- inoculating into the experimental cell the sample to be analyzed after a stabilization time; and
- transferring the data resulting from the measurement to said data acquisition and treatment system.

The stage of extracting from said curve at least one parameter indicative of the behavior of said microorganism can include the division of said curve into at least two sections and the mathematical adjustment of each of said sections to two different mathematical equations, at least one of said sections adjusted to an exponential equation and/or at least another one of said sections is adjusted to a polynomial equation.

Said parameter can be at least one of the following: mathematical function; adjustment parameters of said mathematical function; absolute or relative maximum value or values of said signal; absolute or relative minimum value or values of said signal; recording or detection times of said signal; growth constant; generation time or amount of heat exchanged.

Said microorganisms of interest, generally any microorganism capable of bringing about the development of infection, can be one of the following: *Enterococcus faecalis, Escherichia coli, Proteus mirabilis, Klebsiella pneumoniae, Staphylococcus aureus, Staphylococcus epidermidis, Staphylococcus warneri, Staphylococcus hominis, Pseudomonas aeruginosa* or *Streptococcus pyogenes.*

The present invention provides in a second inventive aspect, an apparatus for detecting and/or identifying a microorganism in a sample, including:

- means suitable for obtaining a database which establishes a correlation between at least one parameter and a plurality of microorganisms, among which said microorganism is included;
- means suitable for analyzing said sample and for supplying an electrical signal proportional to the energy exchanged by said sample;

- means suitable for obtaining a curve which relates said electrical signal with respect to time;
- means suitable for extracting from said curve at least one parameter indicative of the behavior of said microorganism; and
- means suitable for comparing said parameter extracted from said curve with said parameter contained in said database to establish a conclusion as to the presence of said microorganism in said sample.

This apparatus is suitable for performing the process object of the present invention, described above in the first inventive aspect, and presents the already indicated advantages.

According to a preferred embodiment of the present invention, said means suitable for analyzing the sample comprise at least:

a) a Calvet-type heat conduction calorimeter, provided with at least one thermostatic system and with one detector system including two identical thermopiles;

- wherein said detector system further comprises two cells, an experimental cell and a control cell; said experimental cell suitable for receiving a portion of said sample plus a culture medium and said control cell suitable for receiving another portion of culture medium plus physiological saline, preferably 0.9% sodium chloride; and

- wherein both cells are housed in the respective internal enclosures of each of said thermopiles; and

b) a data acquisition and treatment system suitable for receiving the data resulting from the measurement of said sample in said calorimeter.

The data acquisition and treatment system can advantageously include at least,

- a precision multimeter, suitable for collecting the data and signals supplied by said means suitable for analyzing the sample;
- an analog/digital converter; and
- data memory and processing means, for example a computer, suitable for receiving said digital signal, for processing said signal, for obtaining a curve which relates said electrical signal with respect to time and for extracting and storing in a database a plurality of parameters related with said signal and indicative of the behavior of said microorganism.

The means suitable for extracting from said curve at least one parameter indicative of the behavior of said microorganism can also include means suitable for performing the division of said curve into at least two sections and for mathematically adjusting each of said sections to two different mathematical equations, at least one of said sections being adjusted to an exponential equation and/or the other one to a polynomial equation.

The mathematical function resulting from said adjustment is, advantageously, the union of said sections adjusted to said equations, wherein the number of said sections is greater than two, for example a number comprised between three and ten, and wherein at least one of the sections is adjusted to an exponential equation and another one of the sections is adjusted to a polynomial function.

The parameter can be at least one of the following: mathematical function resulting from said adjustment; area under the curve of said mathematical function; adjustment parameters of said mathematical function; absolute or relative maximum value or values of said signal; absolute or relative minimum value or values of said signal; recording or detection times of said signal; growth constant; generation time or amount of heat exchanged.

The technical advantages of each of these particularly advantageous embodiments of the present invention are sufficiently explained in the following text of the present invention patent application, particularly in the section in which an embodiment of the invention is described in detail.

### Brief Description of the Drawings

To better understand the invention, its objects and its advantages, the following drawings are attached to the specification:

Figure 1 shows a detail of the differential assembly of the thermopiles of a Calvet-type microcalorimeter.
Figure 2 depicts the regression line for dynamic calibration at 298.15 k with a calibration constant of C=23.8 J/$\mu$V.h
Figure 3 depicts the regression line for chemical calibration at 309.65 k with a calibration constant of C=23.4 J/$\mu$V.h
Figure 4 depicts the experimental assembly according to an embodiment of the present invention.
Figure 5 depicts a control thermogram
Figure 6 depicts thermograms of the *Staphylococcus aureus* bacterium.
Figure 7 depicts thermograms of the *Staphylococcus epidermidis* bacterium.
Figure 8 depicts thermograms of the *Staphylococcus warneri* bacterium.
Figure 9 depicts thermograms of the *Staphylococcus hominis* bacterium.
Figure 10 depicts thermograms of the *Streptococcus pyogenes* bacterium.
Figure 11 depicts thermograms of the *Enterococcus faecalis* bacterium.
Figure 12 depicts thermograms of the *Escherichia coli* bacterium.
Figure 13 depicts thermograms of the bacterium *Proteus mirabilis*
Figure 14 depicts thermograms of the *Klebsiella pneumoniae* bacterium.
Figure 15 depicts thermograms of the *Pseudomonas aeruginosa* bacterium.
Figure 16 is the graphic representation of the first section of the *E. faecalis* ($10^6$ CFU/ml) thermogram.
Figure 17 is the graphic representation of the second section of the *E. faecalis* ($10^6$ CFU/ml) thermogram.
Figure 18 is the graphic representation of the third section of the *E. faecalis* ($10^6$ CFU/ml) thermogram.
Figure 19 is the graphic representation of the fourth section of the *E. faecalis* ($10^6$ CFU/ml) thermogram.
Figure 20 is the graphic representation of the fifth section of the *E. faecalis* ($10^6$ CFU/ml) thermogram.
Figure 21 is the graphic representation of the sixth section of the *E. faecalis* ($10^6$ CFU/ml) thermogram.
Figure 22 is the graphic representation of the seventh section of the *E. faecalis* ($10^6$ CFU/ml) thermogram.
Figure 23 is the graphic representation of the eighth section of the *E. faecalis* ($10^6$ CFU/ml) thermogram.
Figure 24 is the graphic representation of the ninth section of the *E. faecalis* ($10^6$ CFU/ml) thermogram.
Figure 25 depicts the complete *E. faecalis* ($10^6$ CFU/ml) thermogram.

## Embodiments of the Invention

Microcalorimetry is the experimental technique which allows to determine, with high sensitivity, the energy exhibited as a consequence of any transformation process.

The first known calorimeter is due to Professor of Medicine and Chemistry of the University of Glasgow, Joseph Black, who in 1761 made a hole in the center of a bar of ice, introduced a solid substance of predetermined mass and temperatures, and after waiting for 20 minutes to reach thermal equilibrium, with a previously weighed dry sponge, absorbed the melted water and weighed it again, whereby he knew the mass of melted ice. This is how he developed the concepts of specific heat and latent heat, defining this as the heat necessary for producing changes in state without temperature variations. He had just created the first isothermal calorimeter and initiated calorimetry as a science.

At almost the end of the 19th century, French chemist Pierre Eugène Marcelin Berthelot made significant progress within this new line of research when he invented, in 1881, a calorimetric pump which allowed him to study combustion reactions at a constant volume and in the presence of pressurized oxygen.

Richards, Nobel prize in Chemistry in 1914, developed adiabatic calorimetry according to an idea proposed 50 years earlier by French Scientist Pearson (1849). The purpose was to eliminate the corrections that thermal leaks, which are always difficult to evaluate, made it necessary to introduce in all calorimetric experiments either a hot water stream, or an electrical resistance, or a chemical reaction, considering the last of the three possible methods the most appropriate. However, today it is known that obtaining a total adiabatic state is virtually impossible even with current technical advances.

In that period, evolution of the different types of calorimeters progressed dramatically as a result of the effort of great researchers who dedicated an exceptional amount of ingenuity and patience to building new apparatuses. It was necessary to wait until 1923 for Albert Tian to build the first heat conduction microcalorimeter in Marseilles. It was a device which used the Peltier effect to compensate for exothermal effects, in which stirring in the calorimetric bath was suppressed and which allowed to take micromeasurements.

In 1956, Edouard Calvet, a disciple of Tian, considering Joule's proposal that all calorimeters had to have twin cells and using the idea of heat conduction developed by Tian as a basis, built the first heat flow calorimeter with thermopiles, which today still bear his name, therefore became the person who discovered and drove modern microcalorimetry. The known Calvet calorimeter has not yet be surpassed by the numerous versions which, based on this measurement of the heat flow, have been created by different companies.

A microcalorimeter is formed by an internal enclosure or detector system where the thermal processes to be studied occur (calorimeter per se) and the thermostat surrounding it. The thermal phenomena taking place during a process tend to vary the temperature of the calorimeter, thereby causing energy exchanges between the two enclosures through the medium that separates them, which can be detected by thermoelectric phenomena.

Therefore, the calorimeter is referred to as the internal enclosure (I.E.), and the thermostat surrounding it is referred to as the external enclosure (E.E.). The parameters which characterize a calorimeter and which will determine its operation and design are the heat flow ($\Phi$) established between the internal and external enclosures, heat conductivity ($\Lambda$) of the medium separating them and temperatures ($\theta_{int}$, $\theta_{ext}$) of the internal and external enclosures, respectively.

The heat flow ($\Phi$) between the two enclosures can be determined from Fourier's law:

$$\phi = \Lambda\left(\theta_{int} - \theta_{ext}\right)$$

The greater the difference of temperatures between I.E. and E.E. and the greater the heat conductivity of the medium separating them, the greater the heat flow is between both enclosures.

Calorimeters are classified according to the heat exchange occurring between the calorimeter per se and its surroundings, as a consequence of the difference of temperatures between them.

In adiabatic calorimeters there is no heat transfer between their boundaries ($\Phi=0$) because the internal and external enclosures are separated by a completely adiabatic wall ($\Lambda=0$) referred to as adiabatic shield and theoretically impermeable to the passage of heat.

However, the term adiabatic is used imprecisely because in practice, no calorimeter is truly adiabatic, although heat exchange between the calorimeter and its surroundings can be reduced by minimizing the differences of temperatures between both enclosures, minimizing the heat transfer coefficient or reducing the heat exchange time to a minimum.

In this type of calorimeter, it is the temperature increase or decrease throughout the process that must be measured with the greatest possible precision.

The name isothermal calorimeter encompasses all those calorimeters in which, ideally, there is no temperature change during the experiment. The use of the term isothermal entails constant temperature in any part of the calorimeter over time, i.e., the temperature of both the internal and external enclosures must be virtually the same ($\theta_{int}=\theta_{ext}$), which causes the conductivity of the medium separating them to take very high values ($\Lambda=\infty$) in order to maintain the heat flow Φ=0.

This equipment normally uses a phase change to maintain this constant temperature such that the amount of substance that changes state (solid-liquid, in the case of the Bunsen ice calorimeter, or liquid-vapor) is proportional to the energy released by the process. However, there are other alternative methods, including those using electric heaters (Joule effect) to balance heat absorption, or those using electric cooling (Peltier effect) to compensate for the release thereof.

The basis of heat conduction calorimetry lies in the fact that most of the heat developed in the internal enclosure is taken to the external enclosure by conduction through an association of thermocouples connected in series, such that the electromotive force produced in the system by the Seebeck effect is proportional to the heat flow going through the thermopile.

If it is considered that $\theta_{ext}$ (temperature of the external enclosure) remains constant and that $\theta_{int}$ (temperature of the internal enclosure) varies due to the process performed, the flow produced will vary as a function of time, so clearing up in the preceding equation, conductivity ($\Lambda$) of the medium separating the enclosures can be written as:

$$\Lambda = \frac{\phi}{\theta_{int} - \theta_{ext}}$$

Heat conduction calorimeters precisely measure thermal leaks between the calorimeter and thermostat.

Some of its main features are the capacity to measure heat flow produced between the two enclosures (internal and external) without the need for agitation because it is not based on temperature measurements but rather on the measurement of the electromotive force produced in the welds of the thermocouples surrounding the internal and external enclosures (due to the Seebeck effect), which is proportional to heat flow; the "opposition" (differential) assembly of two identical thermopiles, such that the experimental zero drift due to temperature variations is suppressed; the possibility of performing calorimetry at high and low temperatures, with virtually the same assembly, by simply making minor adjustments in the thermostats, and with no limitation to measurement time; and versatility in the size of the samples to be studied.

A Calvet heat conduction microcalorimeter has been used in the embodiments of the present invention that will be described.

Heat conduction microcalorimetry was developed by Calvet (1956), who greatly transformed and improved a single-thermopile calorimeter devised by Tian (1923). To assure constant temperature, he attempted to cancel out the heat flow between an isolated enclosure and the exterior using a thermoelectric transfer method which accurately compensated for the thermal effects produced inside the internal enclosure. Since the resources at that time did not allow sufficient automation of the calorimeter, in 1958 Calvet proposed considerably increasing heat conduction between the internal enclosure and its surroundings, directly measuring heat transferred by conduction. The system thus created is considerably isothermal. In fact, stabilization of the temperature of the external enclosure formed by a metal calorimetric block with a predetermined geometry allows to obtain great thermal stability due to its multiple enclosures, forming a thermostat which maintains the temperature with a precision of $\pm 0.05$ K, whereby allowing the detection of small heat flows of the order of $10^{-6}$ W.

A Calvet-type microcalorimeter is used in the embodiment of the present invention to determine the heat flow resulting from bacterial metabolism at constant pressure. It is a heat conduction or quasi-isothermal flow calorimeter which allows to record very small heat flows, of the order of a microwatt, proportional to the electromotive force of the thermopile.

This working method has a series of advantages: it allows to perform a direct measurement of the heat of mixing without needing to know the heat capacities of its components or mixtures; calibration is easy and precise; it allows working in a broad temperature range; the speed of the mixing process does not affect the results because the energy developed during said process and the measurement thereof are independent of the time the experiment lasts; and the obtained results are easily reproducible.

The separation between the calorimeter per se and the experimental assembly that is additionally required allows making changes to the latter without interfering in the calorimeter.

The Calvet-type microcalorimeter used in the embodiment of the present invention basically consists of a thermostatic system, a detector system and a data acquisition and treatment system.

The multiple-enclosure thermostat is formed by an iron-sheet cylinder containing insulating material and separating the thermostatic system from the exterior with a thick layer of glass wool and by an aneroid thermostat, formed by five concentric aluminum cylinders separated from one another by layers of asbestos (heat insulator) of the same thickness. Its purpose is to maintain the calorimetric elements at the same temperature because given its arrangement, it allows any thermal effect from the exterior to propagate more readily over the metal layers of the enclosure than through the insulation itself, thus achieving a more homogenous diffusion and enabling conduction towards the bases.

The electrical heating resistance, connected to a contact thermometer controlled by an electronic temperature regulator, is wound outside the set of aluminum cylinders (multiple enclosures).

The detector system is formed by two identical thermopiles arranged symmetrically with respect to a diametral plane and connected in opposition (differential assembly) surrounded by two frustoconical aluminum parts (equitable heat distributing system). The differential assembly allows external disturbances to equally reach both elements, compensating each other and allowing the direct detection of the difference between measured heat flows. One of the thermopiles is used as an "experimental" setting, performing therein the process to be studied, while the other one is always at the temperature of the thermostat and is used as a "control".

Each of the thermopiles is formed by a cylindrical recipient with pure silver walls of little thickness, wherein the cylindrical cell where the calorific phenomenon to be studied takes place is introduced, and which is in turn coaxial with another larger cylinder that is fitted in the frustoconical block. Both cylinders are joined by chromel-constantan thermocouples connected to one another in series, homogenously distributed in a radial manner and insulated by a thin layer of mica, thermally connecting the internal enclosure (silver wall of the internal enclosure) with the external enclosure (aluminum wall of the external enclosure), which is presumably at constant temperature. The number and size of these thermocouples is calculated such that they allow obtaining maximum sensitivity and precision, as well as minimum thermal inertia, heat being transferred through them in either direction by conduction.

Two intertwined similar parts having different functions can be considered in each thermopile: the detecting part, producing a Seebeck effect electromotive force proportional to the power developed in the experimental process, and formed by 800 thermocouples, and the compensating part, formed by 200 thermocouples, which can be connected to an electronic system which regulates and measures current intensity to thus compensate, by the Peltier effect, for the heat absorbed or produced in the experiment.

This difference allows to work at three different degrees of detection sensitivity, which can be selected by means of a switch located in the external part of the calorimeter:

Small sensitivity (S.S.): the compensating part of the thermopile is used as a detector and 200 thermocouples are enabled.

Medium sensitivity (M.S.): detection is performed with the detecting part, activating 600 thermocouples.

Great sensitivity (G.S.): both parts of the thermopile connected in series operate, 800 thermocouples. The embodiments of the present invention described below and the data associated with them were carried out using this degree of sensitivity.

The thermopiles are accessible from the exterior through two cylindrical holes, parallel to one another, spanning from the upper part of the microcalorimeter to the internal enclosure. The large distance separating the thermocouples from the entrance allows minimizing the effects of heat conduction towards the exterior.

Concerning the data acquisition and treatment system, the calorific value developed in the cell is transformed by the thermocouples into electromotive force (Seebeck effect), which is transmitted to a multimeter that collects data with a precision of 0.01%, working in a range of 200 mV. The same multimeter converts the analog signal into a digital signal and transfers it by means of a connector to a computer that collects the information by means of a Qbasic language program. A subroutine is responsible for storing the values in a file and for integrating the curve depicting the output signal over time.

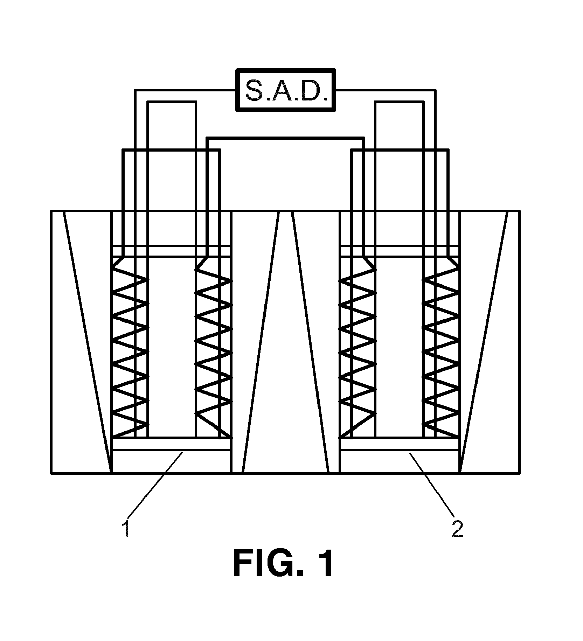

The differential assembly is shown in Figure 1, where the experimental cell 1 and the control cell 2 can be seen. The objective of the differential calorimetric method lies in suppressing accidental temperature variations in the external enclosure occurring when the apparatus had only one isolated microcalorimetric element, as in Tian's calorimeter. The system consists of arranging two identical microcalorimetric elements, in the same heat exchange conditions, joined to one another in opposition and with the same external enclosure. The "experimental" cell 1, where the phenomenon to be studied takes place, will be introduced in one of them, and the "control" cell 2, where a thermal phenomenon with known and adjustable power will occur, will be introduced in the other one.

The main advantage of this assembly is that it does not depend on the external temperature, thus assuring stability of the experimental zero, a drawback which is present in a single-element microcalorimeter. In the latter, it is very difficult to reach zero power state in the cell fundamentally because the temperature of the external enclosure, which affects the external welds of the thermocouples, does not remain constant.

When starting a thermal process in the experimental cell, heat energy, Q, is produced in the internal enclosure. This creates a difference of temperature between both internal and external enclosures of the microcalorimeter, and this variation can be measured through the thermopiles.

The characteristics, constitution, arrangement of elements, operation and measurements (in steady state or in variable state) of the microcalorimeter are generally those which are typical and conventional for a Calvet-type microcalorimeter.

Concerning the calibration of the microcalorimeter, to calculate the heat exchanged throughout an experiment, it is necessary to calculate the value of the integral ∫E(t)dt and that of the calibration constant, C, which will depend both on the microcalorimeter used and on the working sensitivity, which in turn is a function of the number of thermocouples involved. Prior calibration of the apparatus must therefore be done, this possibly being an electric calibration, measuring the heat produced by Joule effect at a resistance with a specific value when an electric current with a known intensity is passed through it either for a long time period (static calibration) or by supplying the same power for a short time period (dynamic calibration).

Calibration can alternatively be either chemical, by means of standard mixtures, the thermal effects of which are perfectly determined and verified in different international laboratories of renowned prestige following IUPAC standards, or carried out by using radioactive standards with long semi-decay periods, which supply a constant heat flow independent of the temperature.

In the particular embodiment described, dynamic calibration by Joule effect is proposed, comparing it to the calibration of a hexane and cyclohexane mixture. The other alternatives are also valid.

In electric calibration by Joule effect, and taking into consideration the duration of the passage of current through the resistance, two types of calibration are distinguished:

Static calibration: this is based on the constant and continuous development of a power inside the cell, a steady flow state between the cell and the calorimetric block through the thermocouples wires being established such that the temperature of both enclosures (internal and external) is maintained constant while the supplied power does not vary. To that end, constant intensity is selected in the EJP 30 generator (SETARAM, Lyon, France) which will be maintained until reaching steady flow state conditions.

The calibration constant can be determined from the power generated in the cell ($W = I^2R$) and the maximum electromotive force, $E_{max}$, measuring the separation from experimental zero by means of a digital multimeter connected to the microcalorimeter.

Dynamic calibration: this is performed in variable state supplying electric power for a short time.

To determine the calibration constant, a calibration cell connected to a source of constant intensity EJP 30 (SETARAM, Lyon, France) is introduced in the experimental thermopile. Once the system reaches thermal stability, after approximately two hours, a current with intensity I is passed through the experimental resistance for a short and determined time (between 10 seconds and 2 minutes) to prevent reaching the steady state, part of this heat energy being dissipated by Joule effect. This energy in turn acts by heating the internal enclosure and causing the generation, in the thermocouples by Seebeck effect, of an electromotive force proportional to the heat put into play. This perfectly stabilized current with a precision of $\pm 10^{-6}$ amperes is supplied by the generator. The passage of current is interrupted before reaching the steady state such that once the maximum value is reached, the electrical output signal of the thermopile decreases exponentially until reaching the prolongation of the initial zero (experimental zero).

At this point, the data acquisition system will allow to know the value of the integral given in the preceding equations which corresponds with the experimental area under the curve and which is proportional to the recorded calorific effect. In addition, the energy supplied by the resistance is determined by Joule's law as ($Q = I^2Rt$), so by equaling both expressions:

$$I^2Rt = C\int_{t_1}^{t_2} E(t)dt$$

Therefore, the value of the constant C, which coincides with the slope of the line resulting from the least squares fitting of the values obtained for the integral at different times of heating, can be calculated.

This is the type of calibration that has been followed in the embodiment of the present invention. The experimental results of the dynamic calibration by Joule effect at 298.15 K, are shown in the following table:

| Area (μV.h) | Q(J) |
|---|---|
| 0.002 | 0.041 |
| 0.0047 | 0.116 |
| 0.0275 | 0.66 |
| 0.0477 | 1.194 |
| 0.0515 | 1.308 |
| 0.176 | 4.09 |

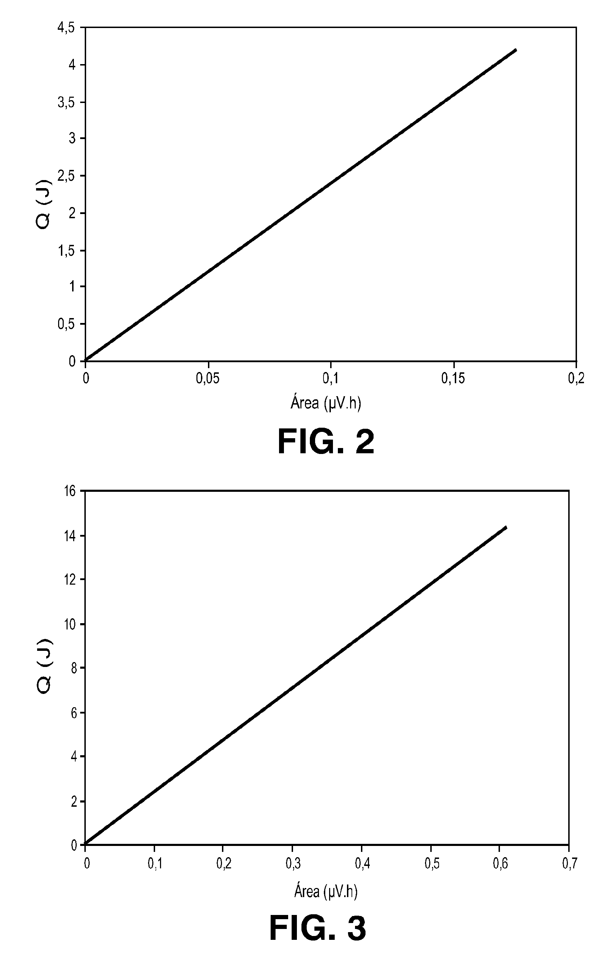

Figure 2 shows the regression line for dynamic calibration at 298.15 k with a calibration constant of C=23.8 J/μV.h

Chemical calibration by means of standard mixtures: The results obtained with the electric calibration by Joule effect are verified by experimentally determining the excess molar enthalpies of the standard system recommended by the IUPAC Commission on Thermodynamics and Thermochemistry, cyclohexane + hexane at 298.5 K and atmospheric pressure.

In this embodiment of the present invention chemical calibration is performed using a hexane and cyclohexane mixture. The working temperature is also 309.65 k.

The results of the chemical calibration are shown in the following table:

| Area (μV.h) | Q(J) |
| --- | --- |
| 0.095 | 2.2 |
| 0.028 | 6.6 |
| 0.61 | 14.3 |

Figure 3 shows the regression line for the chemical calibration a 309.65 k, with a calibration constant of C=23.4 J/μV.h

In the embodiments of the present invention being described, the following bacteria, named in a non-limiting manner, understanding that many other bacteria known by any person skilled in the art are also included in the object of the present invention are used:

- ■ *Staphylococcus aureus*
- ■ *Staphylococcus epidermidis*
- ■ *Staphylococcus warneri*
- ■ *Staphylococcus hominis*
- ■ *Streptococcus pyogenes*
- ■ *Enterococcus faecalis*
- ■ *Escherichia coli*
- ■ *Klebsiella pneumoniae*
- ■ *Proteus mirabilis*
- ■ *Pseudomonas aeruginosa*

## Preparation of the bacterial samples

To determine the microcalorimetric behavior of a bacterial species it is necessary to study samples of the same strain at different concentrations.

In all cases, a pure strain of the bacterium to be studied was used; this strain is inoculated in a blood agar plate and is incubated at 309.65 K in a chamber for 24 hours.

A suspension of bacteria in 0.9% sodium chloride, the concentration of which is adjusted to that corresponding to 0.5 on the McFarland scale, is prepared from the blood agar plate where multiple colonies have grown. Dilutions are then made with 0.9% sodium chloride until obtaining samples which allow to obtain final concentration of $10^6$, $10^5$, $10^3$ or 10 CFU/ml.

Soybean-casein digest broth is used as a culture medium, a non-selective medium in which most pathogenic microorganisms present growth.

## Experimental assembly and data collection

The experiments shown in this embodiment of the present invention were performed in a Calvet-type microcalorimeter, having two aluminum and Teflon cells (experimental and control) with a 10 ml capacity.

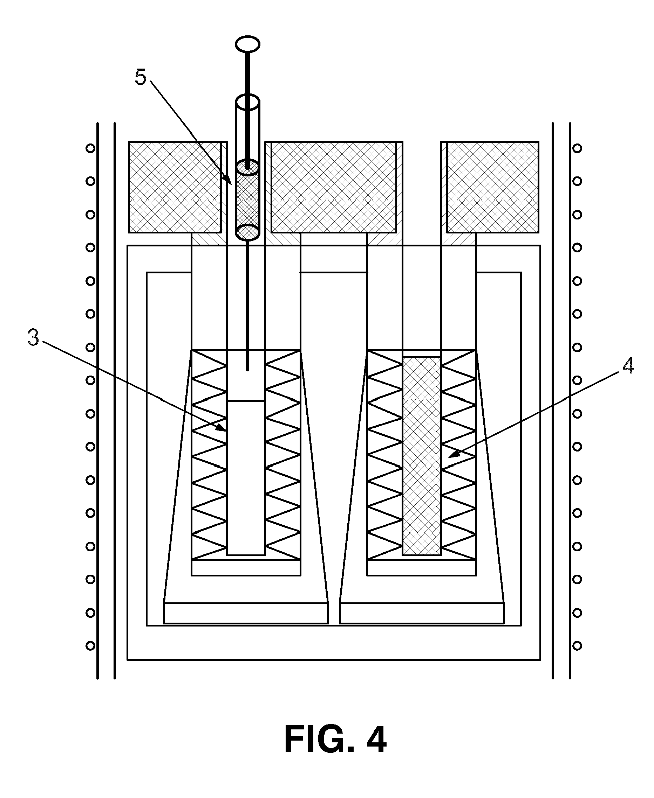

7 ml of culture medium plus 1 ml of 0.9% sodium chloride are incorporated in the control cell, and 7 ml of culture medium are incorporated in the experimental cell. Both cells bind to hollow plastic tubes which will allow to introduce the two cells through two cylindrical holes parallel to one another spanning from the upper part of the microcalorimeter to the internal enclosure of the thermopiles (see Figure 4). The large distance separating the cells from the entrance therefore allows to minimize the effects of heat conduction towards the exterior.

In the experimental assembly according to an embodiment of the present invention of Figure 4, the sample 5, the culture medium 3 and 0.9% sodium chloride and the culture medium 4 can be distinguished.

In the case of the experimental cell, the syringe containing the bacteria inoculum is connected through the Teflon lid with a needle.

After the stabilization time, approximately 2 hours, the culture medium is inoculated with the sample provided in the syringe, using to that end a metal bar with a diameter less than that of the hollow tube to which the experimental cell is joined.

From the time of inoculation, the data acquisition system starts to collect data at a 15-second interval during the time the experiment lasts through a Qbasic language program.

All the samples studied in the microcalorimeter are subject to pH control before and after each experiment.

## Bacterial thermograms

Microcalorimetry is an analytical technique for measuring heat produced or consumed by chemical reactions or changes in physical state, including heat generated during complex biological processes.

All organisms produce heat as a consequence of their metabolism. Heat rate is a suitable measurement of metabolic activity of organisms and their constituent parts, cells and subcellular levels.

When analyzing the microcalorimetric behavior of a bacterial culture a series of electromotive force recordings are obtained which correspond to the energy exchange occurring during the culture period. This energy exchange is dependent on the concentration of the initial inoculum and on the bacterial species. This microcalorimetric behavior is defined by a series of maximums and minimums the value and recording time of which form the growth curve of each bacterial species.

By representing the calorific value difference recorded by the data acquisition system during the experiment over time, a thermogram which depicts the growth curve of each bacterium is obtained.

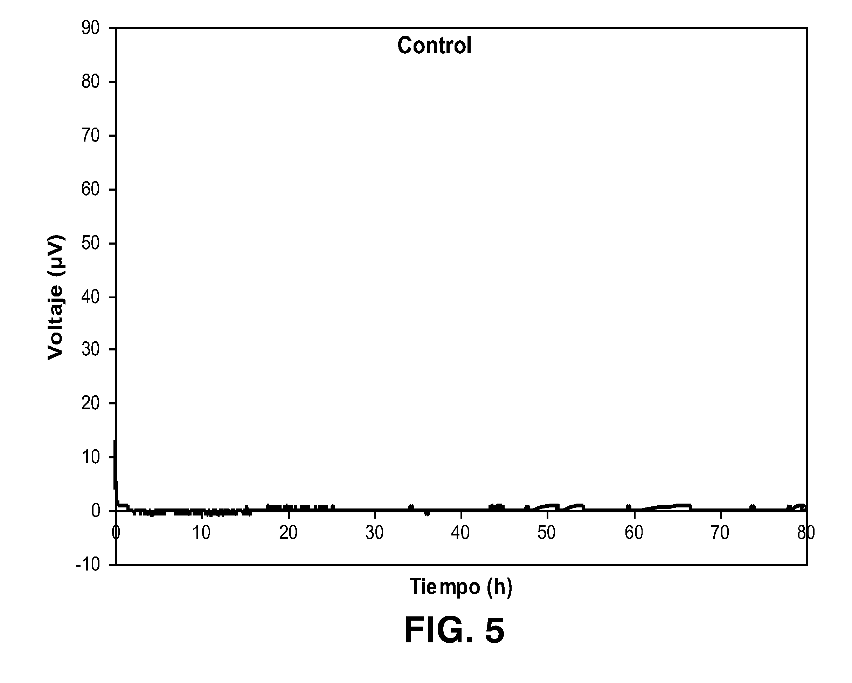

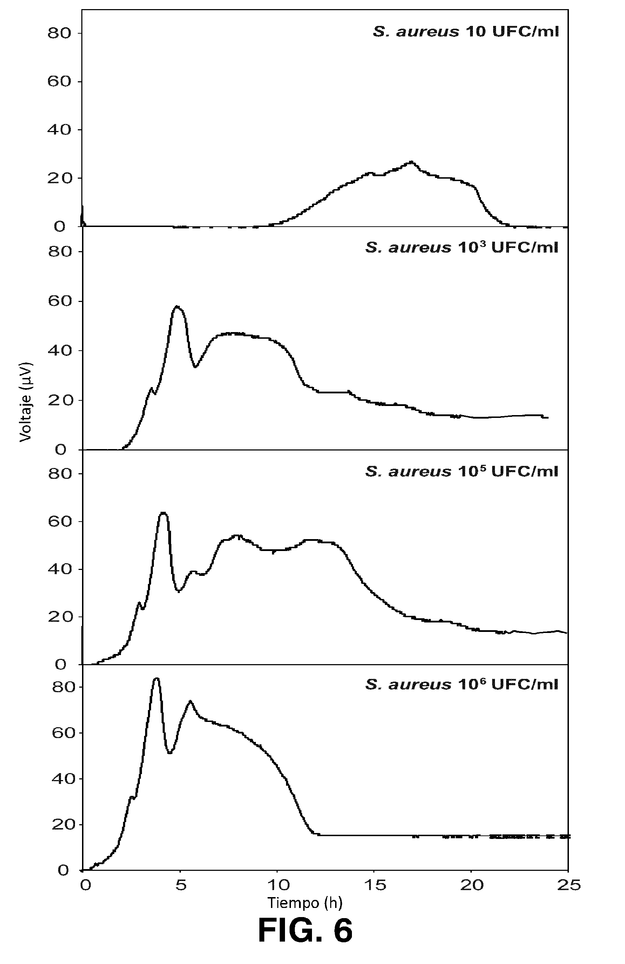

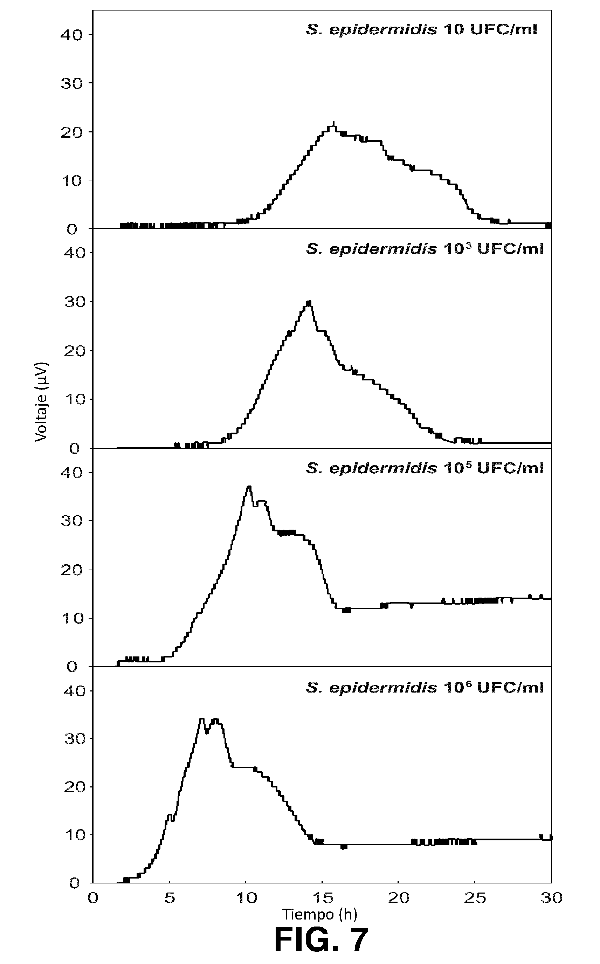

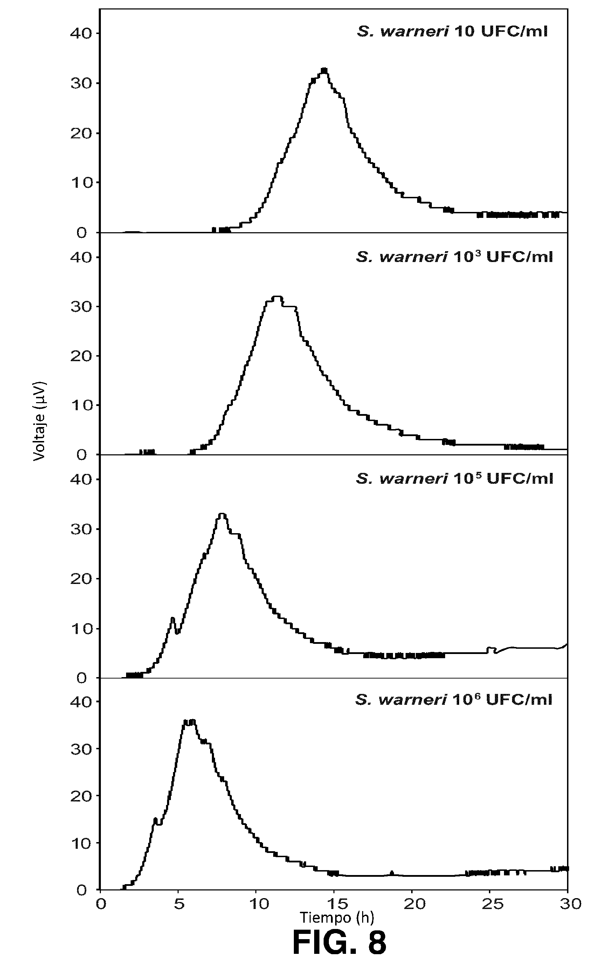

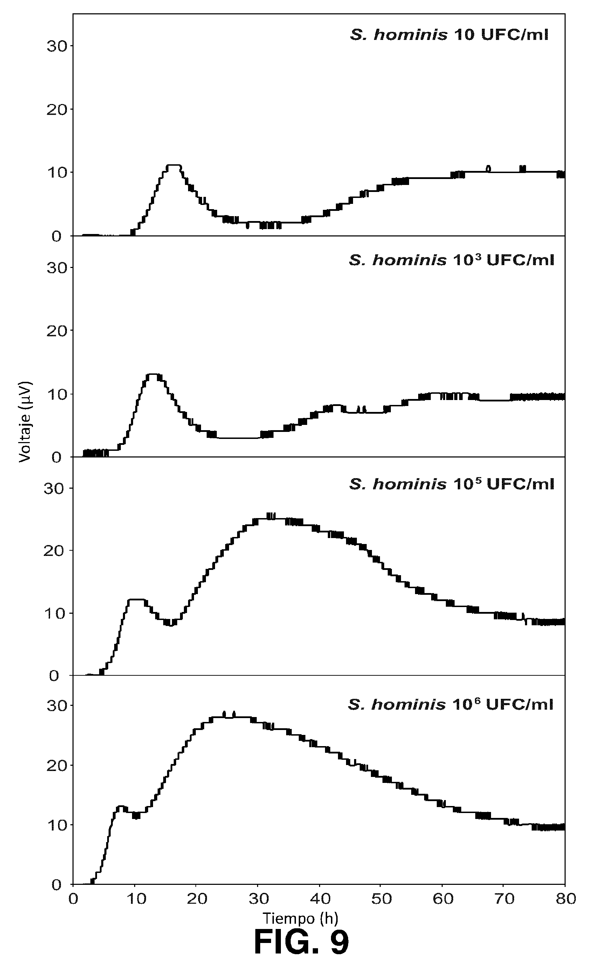

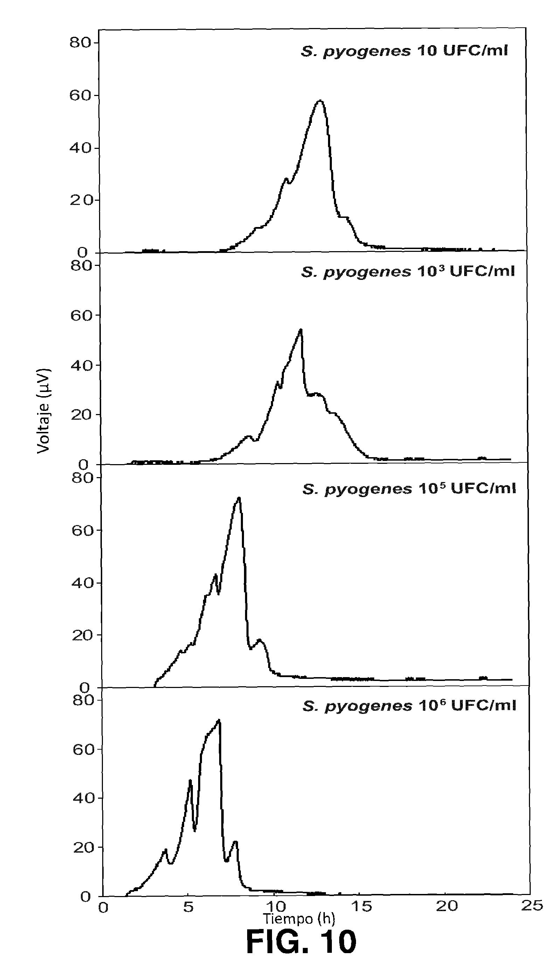

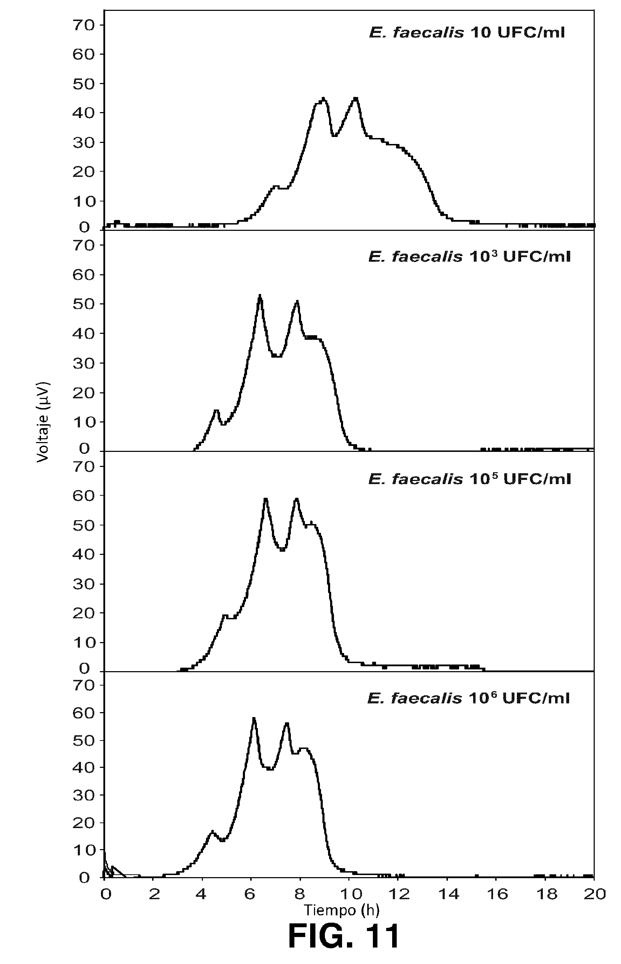

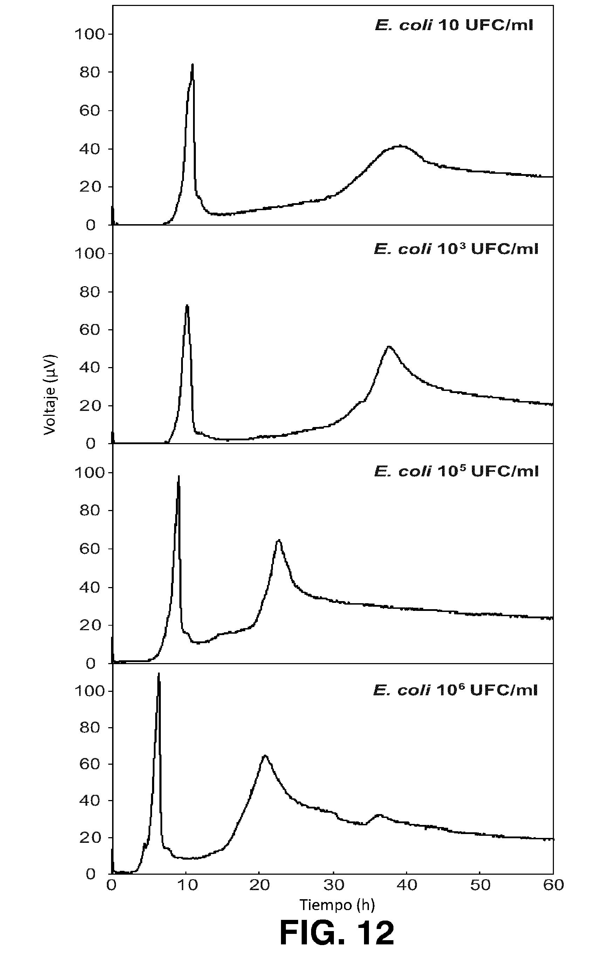

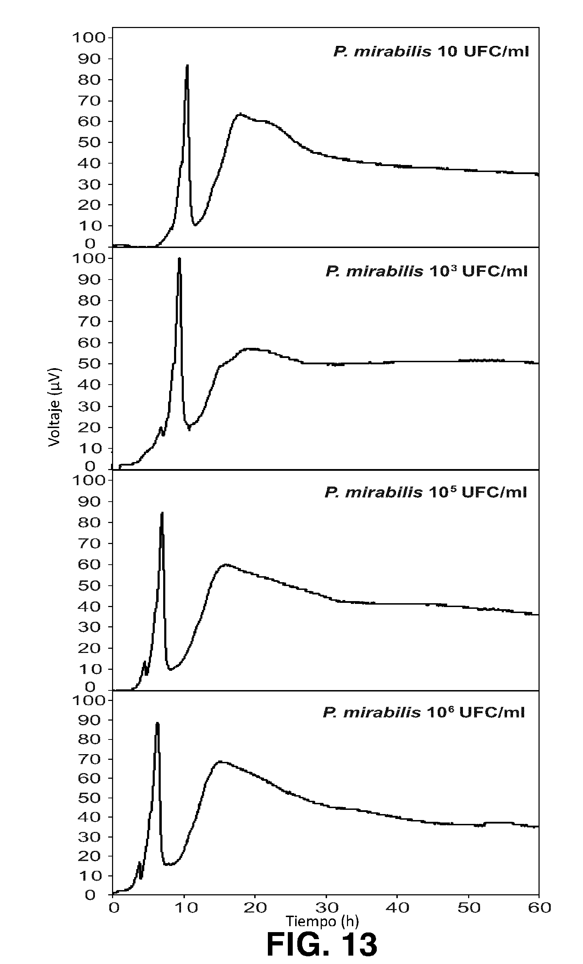

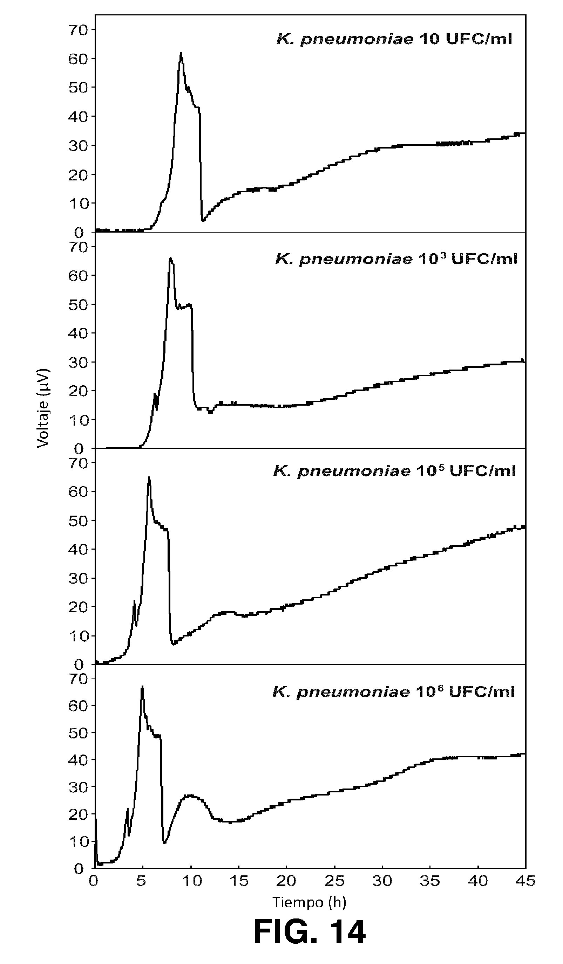

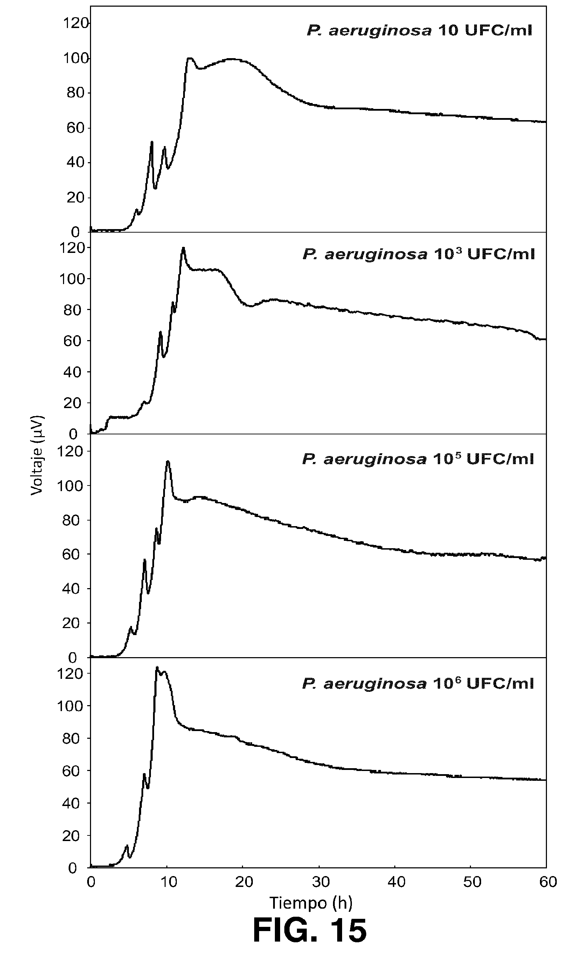

Figures 5 to 15 show both the control thermogram and the thermograms associated with the embodiment of the present invention being described, relative to the aforementioned bacteria.

## Mathematical treatment of the thermograms

The thermograms obtained for each of the bacteria at the different concentrations studied can be mathematically treated. These thermograms are divided into sections and each part is then adjusted to the corresponding mathematical equation.

The different parts of the thermogram are adjusted to exponential equations in the rising and falling phase of each EMF peak

$$y = Ae^{Bx}$$

and to polynomial equations in the areas of greater activity of the thermogram.

$$y = \sum_{i=0}^{n} C_i x^i$$

As an embodiment of the present invention, and understanding that for the rest of the bacteria the process is similar, *E. faecalis* $10^6$ CFU/ml thermogram is described below. The method used can be applied to the thermogram of any bacterium studied by microcalorimetry. In this case, and by way of example, the *E. faecalis* $10^6$ CFU/ml thermogram has been fractioned into 9 sections which are adjusted to respective mathematical equations.

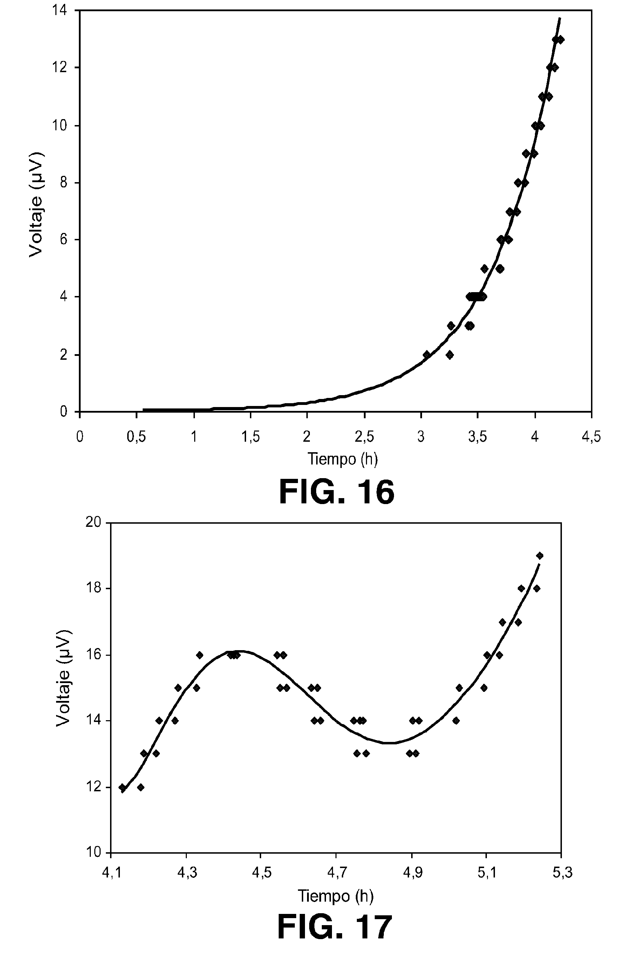

The first phase of the thermogram is adjusted to and exponential-type equation and its graphic representation can be seen in Figure 16.

$$y = 9.6e^{1.721} \qquad R^2 = 0.9684$$

The second phase of the *E. faecalis* thermogram is adapted to a polynomial equation and describes the first EMF peak (see Figure 17).

$$y = 192.5x^6 - 55.12 \cdot 10^{-2} x^5 + 65.65 \cdot 10^{-3} x^4 - 41.61 \cdot 10^{-4} x^3 + 148.1 \cdot 10^{-4} x^2 - 28.04 x + 22.07$$

$$R^2 = 0.927$$

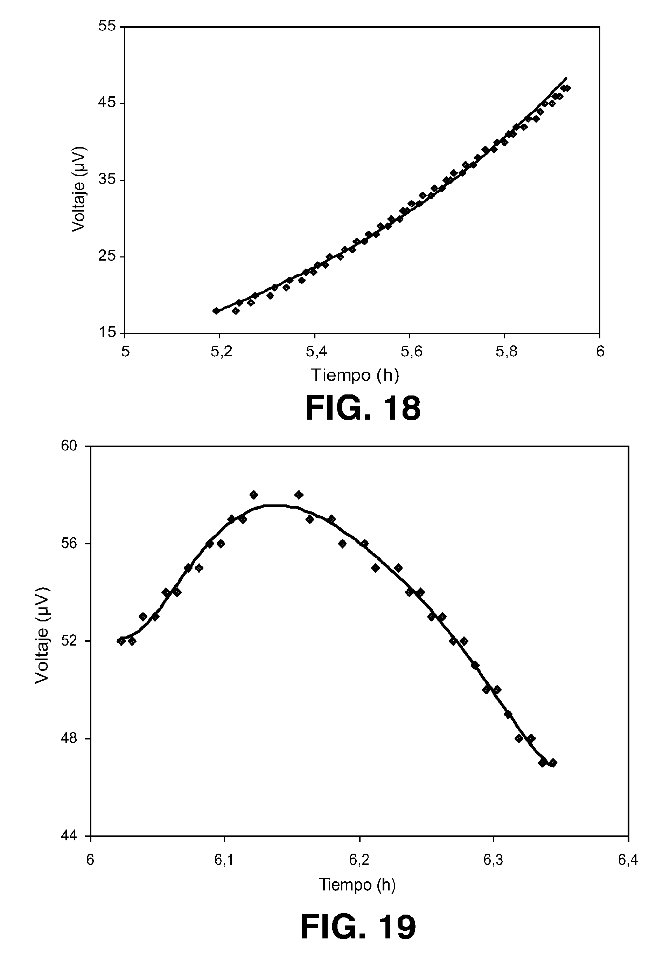

The third phase of the thermogram is adjusted to an exponential equation and its representation is seen in Figure 18.

$$y = 0.016e^{1.352} \quad R^2 = 0.9684$$

The fourth phase of the E. *faecalis* thermogram is defined by another polynomial equation, and its representation is seen in Figure 19.

$$y = 388.3 \cdot 10^{-3} x^6 - 144.4 \cdot 10^{-4} x^5 + 223.7 \cdot 10^{-6} x^4 - 184.9 \cdot 10^{-7} x^3 + 859.3 \cdot 10^{-7} x^2 - 212.9 \cdot 10^{-8} x + 219.9 \cdot 10^{-8}$$

$$R^2 = 0.9896$$

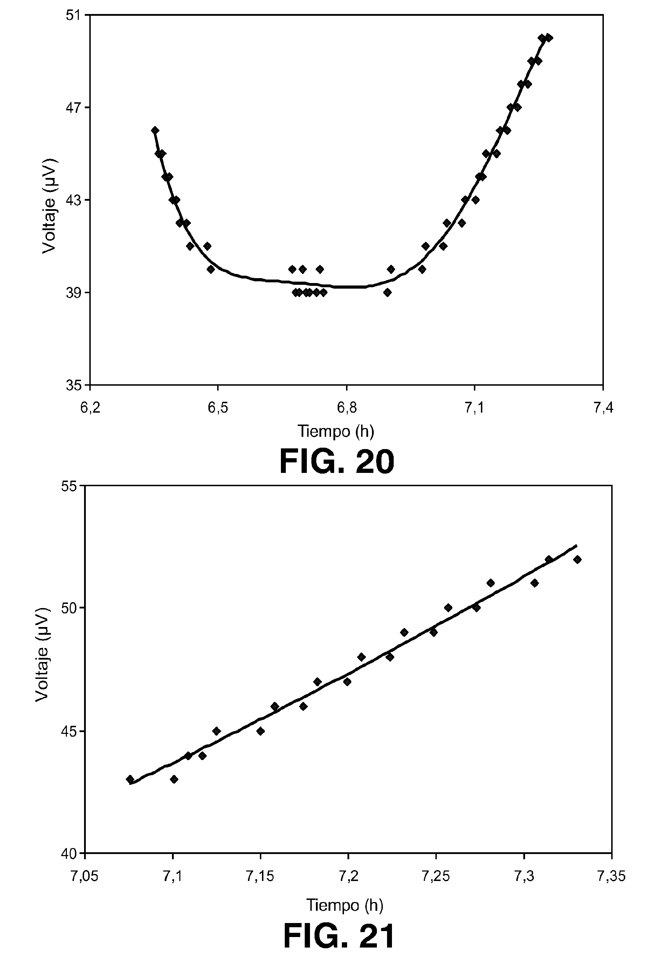

The fifth phase is also adjusted to a polynomial-type equation, the representation of which is seen in Figure 20.

$$y = 75.09 x^6 - 3496.9 x^5 + 669.1 x^4 - 675.9 \cdot 10^{-3} x^3 + 379.5 \cdot 10^{-3} x^2 - 112.9 \cdot 10^{-5} x + 138.9$$

$$R^2 = 0.9949$$

The sixth section of the *E. faecalis* thermogram is adjusted to an exponential equation and its graphic representation is seen in Figure 21.

$$y = 0.145e^{0.804} \quad R^2 = 0.9865$$

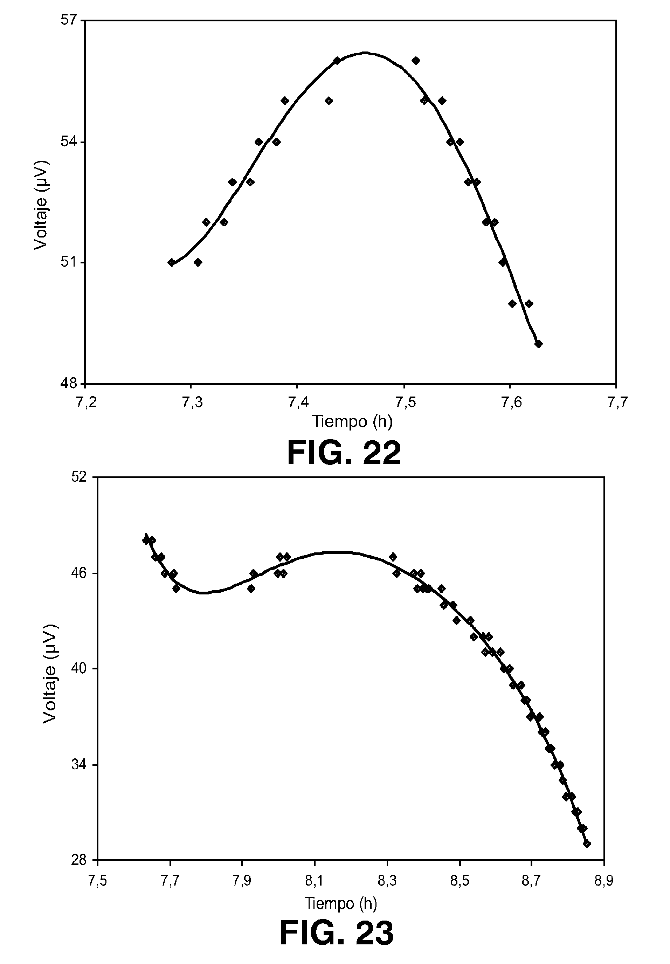

The following phase of the *E. faecalis* thermogram is adjusted to an exponential equation. Its representation is seen in Figure 22.

$$y = 3.159 \cdot 10^{-3} x^4 - 9.461 \cdot 10^{-4} x^3 + 10.62 \cdot 10^{-5} x^2 - 5.298 \cdot 10^{-5} x + 0.9628$$

$$R^2 = 0.9928$$

The eighth section of the thermogram was also adjusted to a polynomial equation, the representation of which is seen in Figure 23.

$$y = 22.75 x^6 - 12.24 \cdot 10^{-2} x^5 + 272.9 \cdot 10^{-2} x^4 - 322.9 \cdot 10^{-3} x^3 + 213.9 \cdot 10^{-4} x^2 - 753.5 \cdot 10^{-4} x + 110.2$$

$$R^2 = 0.9949$$

The last phase of the *E. faecalis* thermogram is adjusted to an exponential equation. Its representation is shown in Figure 23.

$$y = 26.99 \cdot 10^{-13} e^{-3.62} \quad R^2 = 0.9865$$

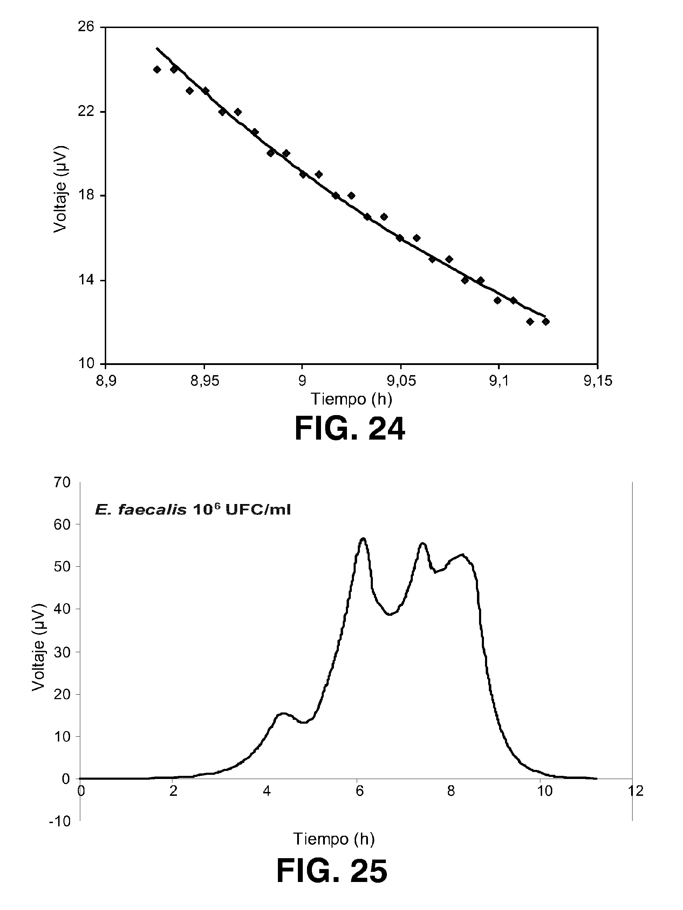

The graphic representation of the nine equations described above form the *E. faecalis* $10^6$ CFU/ml thermogram that can be seen in Figure 24.

The reader understands that the division of the thermogram into nine different equations is only a particular embodiment of one of the stages of the process of the present invention, and that any other number of divisions and/or of different equations can also be done depending on the microorganism studied and on the result of the original curve by simply performing a process of adapting to the circumstances of each case by means mere experimentation.

**Thermal analysis**

A thermogram, characterized by a series of maximum and minimum EMF points which define the behavior of a certain bacterial species in a defined culture medium, is obtained from the microcalorimetric analysis of a bacterial culture medium. The analysis of the bacterial growth curve obtained from the recording of the difference of potential during the culture period allows to obtain parameters characteristic of each bacterial species.

Taking into account the study conditions in which it has been performed the microcalorimetric analyses of the 10 bacterial species on which the present invention is based, it has been accepted that the activity in the thermogram starts when the difference of potential is equal to 2 μV.

Furthermore, as explained above, the areas of greater activity of the thermogram are adjusted to polynomial-type equations. Therefore, the first derivative of these equations allows to know the value of the maximum EMF peaks and the times at which they are recorded.

In the logarithmic phase of the growth curve, n0 is the number of cells at time 0, and nt the number of cells at time t, therefore:

$$n_t = n_0 e^{(kt)}$$

where k is the growth constant.

If $P_W$ is considered to be the energy given off by each cell, then:

$$n_t P_w = n_0 P_w e^{(kt)}$$

Taking into account that $P_0$ *is* the energy given off at t zero and $P_t$ the one given at time t:

$$P_t = P_0 e^{(kt)}$$

$$\ln P_t = Kt + \ln P_0$$

Therefore, the growth constant can be obtained from the exponential equation of adjustment of the rising phase of an electromotive force (EFM) peak of the thermogram of a bacterium.

Generation time (G) is defined as the time it takes a population to double its number and is expressed as:

$$G = \frac{(\ln 2)}{k}$$

The area under the curve (AUC) of any thermogram for a defined time can be calculated using the trapezoidal method. The value of the amount of heat exchanged during a certain time in the culture medium can be obtained by multiplying AUC by C (calibration constant), calculated in this case by means of an electric calibration by Joule effect and verified by means of a chemical calibration with a hexane and cyclohexane mixture.

$$Q = AUC(\mu V \cdot h) \cdot C\left(\frac{J}{\mu V \cdot h}\right)$$

Accordingly, the value of the growth constant, the generation time of a certain bacterium and the amount of heat exchanged in a culture medium can be known in a rapid and simple manner from the thermograms obtained by microcalorimetry.

The tables shown below include the main parameters of the growth curves obtained by microcalorimetry for *E. faecalis.*

For each bacterium:

1. The first table shows the time until the detection of the signal, maximum voltages and the times at which they are recorded. The first maximum peak considered is characterized by having moderate power with respect to later peaks, but it is highlighted as it is characteristic of all the thermograms

2. The second table shows the value of the growth constant and the time of generation of each phase of the thermogram. It shows K and G, the growth constant and the time of generation, respectively, of the maximum voltage peaks. Likewise, the remaining growth constants and times of generation which can be extracted from the graph are marked with an apostrophe "'"relating to their position in the thermogram,.

3. The third table compiles the AUC (area under the curve)

$$Q = AUC(\mu V \cdot h) \times C^* \left( \frac{J}{\mu V \cdot h} \right)$$

and Q (accumulated heat) in the first 24 hours of culture.

Table 1. Time of detection of the signal ($t_d$), first, second, third and fourth of maximum voltage peak ($V_{p1}$, $V_{p2}$, $V_{p3}$, $V_{p4}$) and times of recording ($t_{p1}$, $t_{p2}$, $t_{p3}$, $t_{p4}$), of *E. faecalis*

| Conc. | $t_d$ | $t_{p1}$ | $V_{p1}$ | $t_{p2}$ | $V_{p2}$ |
|---|---|---|---|---|---|
| (CFU/m) | (h) | (h) | ($\mu$V) | (h) | ($\mu$V) |
| $10^6$ | 2.67 | 4.43 | 16 | 6.13 | 58 |
| $10^5$ | 3.32 | 4.99 | 19 | 6.61 | 59 |
| $10^3$ | 3.63 | 4.55 | 13 | 6.37 | 53 |
| 10 | 4.72 | 6.97 | 15 | 8.98 | 45 |

| Conc. | $t_{p3}$ | $V_{p3}$ | $t_{p4}$ | $V_{p4}$ |
|---|---|---|---|---|
| (CFU/m) | (h) | ($\mu$V) | (h) | ($\mu$V) |
| $10^6$ | 7.46 | 56 | 8.14 | 47 |
| $10^5$ | 7.87 | 59 | 8.49 | 51 |
| $10^3$ | 7.88 | 51 | 8.59 | 39 |
| 10 | 10.27 | 45 | 11.36 | 31 |

Table 2. Growth constant (K', K, K") and time of generation (G', G, G") of *E. faecalis*

| Conc. | K' | G' | $R^2$ | K | G | $R^2$ |
|---|---|---|---|---|---|---|
| (CFU/ml) | (h$^{-1}$) | (h) | | (h$^{-1}$) | (h) | |
| $10^6$ | 1.7208 | 0.4028 | 0.9684 | 1.3518 | 0.5128 | 0.9955 |
| $10^5$ | 1.9813 | 0.3498 | 0.9579 | 1.1075 | 0.6259 | 0.9963 |
| $10^3$ | 3.0279 | 0.2289 | 0.9625 | 1.4104 | 0.4915 | 0.9908 |
| 10 | 1.1972 | 0.5789 | 0.9160 | 1.0133 | 0.6840 | 0.9913 |

| Conc. | K" | G" | $R^2$ |
|---|---|---|---|
| (CFU/ml) | (h$^{-1}$) | (h) | |
| $10^6$ | 0.8036 | 0.7055 | 0.9825 |
| $10^5$ | 0.8415 | 0.8237 | 0.9656 |

(continued)

| Conc. | K" | G" | $R^2$ |
|---|---|---|---|
| (CFU/ml) | $(h^{-1})$ | (h) | |
| $10^3$ | 0.8493 | 0.8161 | **0.9839** |
| 10 | **0.4671** | **1.4839** | **0.9680** |

(*) Due to limited data in this phase of the thermogram, neither the growth constant nor the time of generation corresponding to the fourth EMF peak could be extracted

Table 3. $AUC_{24}$ and heat exchanged in the culture *E. faecalis* during the first 24 hours of culture

| Conc. | $AUC_{24}$ | $Q_{24}$ |
|---|---|---|
| (CFU/ml) | $(\mu V.h)$ | (kJ) |
| $10^6$ | 188.6361 | **4.414** |
| $10^5$ | 203.8 | **4.769** |
| $10^3$ | 175.4822 | **4.106** |
| 10 | **219.4869** | 5.136 |

## Claims

1. A method of detecting and/or identifying a microorganism in a sample, including the following stages:

   - obtaining a database which establishes a correlation between at least one parameter and a plurality of microorganisms, among which said microorganism is included;
   - analyzing said sample in a calorimeter which supplies an electrical signal proportional to the energy exchanged by said sample;
   - obtaining a curve which relates said electrical signal with respect to time;
   - extracting from said curve at least one parameter indicative of the behavior of said microorganism; and
   - comparing said parameter extracted from said curve with said parameter contained in said database to establish a conclusion as to the presence of said microorganism in said sample.

2. The method according to claim 1, wherein the stage of obtaining a database consists of performing the following steps:

   - preparing a sample of one of the microorganisms included in said plurality of microorganisms;
   - analyzing said sample in a calorimeter supplying an electrical signal proportional to the energy exchanged by said sample;
   - obtaining a curve which relates said electrical signal with respect to time;
   - extracting from said curve at least one parameter indicative of the behavior of said microorganism;
   - transferring the results of said parameter or parameters together with the identification of the chosen microorganism to an electronic system capable of creating a database which establishes a relationship between both; and
   - repeating all the aforementioned steps with each of the microorganisms included in said plurality of microorganisms.

3. The method according to claims 1 or 2, wherein said stage or stages of analyzing the sample in a calorimeter comprises performing the following steps:

   - providing a Calvet-type heat conduction calorimeter, provided with at least one thermostatic system and with one detector system formed by two identical thermopiles (5),
   - providing a data acquisition and treatment system;
   - providing two cells, an experimental cell (1) and a control cell (2);
   - introducing a portion of culture medium (5) into the experimental cell (1, 3) and introducing a portion of physiological saline, preferably 0.9% sodium chloride, plus culture medium in the control cell;

- introducing both cells in the respective internal enclosures of each of said thermopiles (5);
- inoculating into the experimental cell (1) the sample to be analyzed after a stabilization time; and
- transferring the data resulting from the measurement to said data acquisition and treatment system.

4. The method according to any of claims 1 to 3, wherein the stage of extracting from said curve at least one parameter indicative of the behavior of said microorganism includes the division of said curve into at least two sections and the mathematical adjustment of each of said sections to two different mathematical equations.

5. The method according to claim 4, wherein at least one of said sections is adjusted to an exponential equation.

6. The method according to claims 4 or 5, wherein at least one of said sections is adjusted to a polynomial equation.

7. The method according to any of the preceding claims, wherein said parameter is at least one of the following: mathematical function; adjustment parameters of said mathematical function; absolute or relative maximum value or values of said signal; absolute or relative minimum value or values of said signal; recording or detection times of said signal; growth constant; time of generation or amount of heat exchanged.

8. The method according to any of the preceding claims, wherein said microorganism is selected from at least one of the following: *Enterococcus faecalis, Escherichia coli, Proteus mirabilis, Klebsiella pneumoniae, Staphylococcus aureus, Staphylococcus epidermidis, Staphylococcus warneri, Staphylococcus hominis, Pseudomonas aeruginosa* or *Streptococcus pyogenes*

9. An apparatus for detecting and/or identifying a microorganism in a sample, including:

- means suitable for obtaining a database establishing a correlation between at least one parameter and a plurality of microorganisms, among which said microorganism is included;
- means suitable for analyzing said sample and for supplying an electrical signal proportional to the energy given off by said sample;
- means suitable for obtaining a curve which relates said electrical signal with respect to time;
- means suitable for extracting from said curve at least one parameter indicative of the behavior of said microorganism; and
- means suitable for comparing said parameter extracted from said curve with said parameter contained in said database to establish a conclusion as to the presence of said microorganism in said sample.

10. The apparatus according to claim 9, wherein said means suitable for analyzing the sample comprise at least:

a) a Calvet-type heat conduction calorimeter, provided with at least one thermostatic system and with one detector system including two identical thermopiles (5);

- wherein said detector system further comprises two cells, an experimental cell (1) and a control cell (2); said experimental cell (1, 3) suitable for receiving a portion of said sample plus culture broth (5) and said control cell (2, 4) suitable for receiving another portion of culture medium plus physiological saline, preferably 0.9% sodium chloride; and
- wherein both cells are housed in the respective internal enclosures of each of said thermopiles (5); and

b) a data acquisition and treatment system suitable for receiving the data resulting from the measurement of said sample in said calorimeter.

11. The apparatus according to claim 10, wherein said data acquisition and treatment system includes at least

- a precision multimeter suitable for collecting the data and signals supplied by said means suitable for analyzing the sample;
- an analog/digital converter; and
- data memory and processing means, for example a computer, suitable for receiving said digital signal, for processing said signal, for obtaining a curve which relates said electrical signal with respect to time and for extracting and storing in a database a plurality of parameters related with said signal and indicative of the behavior of said microorganism.

**12.** The apparatus according to any of claims 9 to 11, wherein said means suitable for extracting from said curve at least one parameter indicative of the behavior of said microorganism include means suitable for performing the division of said curve into at least two sections and for mathematically adjusting each of said sections to two different mathematical equations.

**13.** The apparatus according to claim 12, wherein at least one of said sections is adjusted to an exponential equation.

**14.** The apparatus according to claims 12 or 13, wherein at least one of said sections is adjusted to a polynomial equation.

**15.** The apparatus according to claims 12 to 14, wherein the mathematical function resulting from said adjustment is the union of said sections adjusted to said equations, wherein the number of said sections is greater than two, for example a number comprised between three and ten, and wherein at least one of the sections is adjusted to an exponential equation and another one of the sections is adjusted to a polynomial function.

**16.** The apparatus according to any of claims 9 to 15, wherein said parameter is at least one of the following: mathematical function resulting from said adjustment; area under the curve of said mathematical function; adjustment parameters of said mathematical function; absolute or relative maximum value or values of said signal; absolute or relative minimum value or values of said signal; recording or detection times of said signal; growth constant; generation time or amount of heat exchanged.

**FIG. 1**

FIG. 2

FIG. 3

**FIG. 4**

EP 2 623 967 A1

**FIG. 5**

FIG. 6

**FIG. 7**

FIG. 8

**FIG. 9**

**FIG. 10**

**FIG. 11**

**FIG. 12**

FIG. 13

FIG. 14

FIG. 15

FIG. 16

FIG. 17

FIG. 18

FIG. 19

FIG. 20

FIG. 21

FIG. 22

FIG. 23

**FIG. 24**

**FIG. 25**

Europäisches
Patentamt

European
Patent Office

Office européen
des brevets

# EUROPEAN SEARCH REPORT

Application Number

EP 12 38 2043

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOPEZ D ET AL: "ANALYSIS OF MICROCALORIMETRIC CURVES FOR BACTERIAL IDENTIFICATION", CANADIAN JOURNAL OF MICROBIOLOGY, NRC RESEARCH PRESS, CA, vol. 33, no. 1, 1 January 1987 (1987-01-01), pages 6-11, XP009076029, ISSN: 0008-4166 * the whole document * ----- | 1-16 | INV. G01N25/20 C12Q1/04 |
| X,D | WO 2007/010379 A2 (UNIV BASEL [CH]; UNIV HOSPITAL OF BASEL [CH]; DANIELS ALMA U [CH]; WIR) 25 January 2007 (2007-01-25) * page 3, last paragraph - page 4, paragraph 1 * * page 6, paragraph 2 * * page 7, paragraph 3 * * page 9, paragraph 2 - page 10, paragraph 1 * * page 14, paragraph 2 * * page 16, last paragraph * * page 18, paragraph 1 * * page 21, paragraphs 2,3 * * page 23, paragraphs 4,5 * * page 28, last paragraph * * page 29, paragraph 2 * ----- | 1,2,7-9, 16 | |

TECHNICAL FIELDS
SEARCHED (IPC)

G01N
C12Q

The present search report has been drawn up for all claims

| Place of search | Date of completion of the search | Examiner |
|---|---|---|
| Munich | 25 June 2012 | Filipas, Alin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 12 38 2043

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2012

| Patent document cited in search report | | Publication date | Patent family member(s) | | Publication date |
|---|---|---|---|---|---|
| WO 2007010379 | A2 | 25-01-2007 | EP | 1913150 A2 | 23-04-2008 |
| | | | US | 2009317859 A1 | 24-12-2009 |
| | | | WO | 2007010379 A2 | 25-01-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3552207 A
- US 3765237 A
- US 20040235086 A

- WO 2007010379 A
- EP 1785722 A

**Non-patent literature cited in the description**

- **THOMAS MASKOW et al.** Calorimetric real time monitoring of lambda prophage induction. *Journal of Virological Methods,* 2010, vol. 168, 126-132
- ''The heat is on'': Rapid microcalorimetric detection of mycobacteria in culture. *Tuberculosis,* 2010, vol. 90, 57-59
- **DAI CHUNMEI et al.** Investigation of anti-microbial activity of catechin on Escherichia coli growth by microcalorimetry. *Environmental Toxicology and Pharmacology,* 2010, vol. 30, 284-288
- **WEIJUN KONG et al.** *Screening for novel antibacterial agents based on the activities of compounds on metabolism of Escherichia coli: a microcalorimetric study*

- **G. BUTTIGLIERI et al.** Microcalorimetry: A tool to investigate aerobic, anoxic and anaerobic autotrophic and heterotrophic biodegradation. *Biochemical Engineering Journal,* 2010, vol. 52, 25-32
- **F. ALLENBERGER.** Listeria Monocytogenes - Microcalorimetric Investigations Regarding The Antibacterial Efficiency of Chemotherapeutics. *Thermochimica Acta,* 1987, vol. 119, 113-119
- **XIE CHANG-LI et al.** Microcalorimetric Study Of Bacterial Growth. *Thermochimica Acta,* 1988, vol. 123, 33-41
- **NATIVIDAD LAGO RIVERO et al.** Microcalorimetric study of the growth of Enterococcus faecalis in an enriched culture medium. *J Therm Anal Calorim,* 2011